(19) Europäisches Patentamt
European Patent Office
Office européen des brevets

(11) EP 1 001 790 B1

(12) EUROPEAN PATENT SPECIFICATION

(45) Date of publication and mention of the grant of the patent:
**29.09.2004 Bulletin 2004/40**

(21) Application number: **98934085.6**

(22) Date of filing: **13.07.1998**

(51) Int Cl.[7]: **A61K 31/715**, A61K 9/127

(86) International application number:
**PCT/SE1998/001371**

(87) International publication number:
**WO 1999/003481 (28.01.1999 Gazette 1999/04)**

# (54) COMPOSITIONS FOR LUBRICATING AND SEPARATING TISSUES AND BIOLOGICAL MEMBRANES

ZUSAMMENSETZUNG ZUR SCHMIERUNG UND TRENNUNG VON GEWEBEN UND BIOLOGISCHEN MEMBRANEN

COMPOSITION DESTINEE A LA LUBRIFICATION ET A LA SEPARATION DE TISSUS ET DE MEMBRANES BIOLOGIQUES

(84) Designated Contracting States:
**AT BE CH CY DE DK ES FI FR GB GR IE IT LI LU MC NL PT SE**

(30) Priority: **14.07.1997 SE 9702698**

(43) Date of publication of application:
**24.05.2000 Bulletin 2000/21**

(73) Proprietors:
- **Bengmark, Stig**
  **S-220 05 Lund (SE)**
- **LARSSON, Kare**
  **S-237 00 Bjärred (SE)**
- **Lindman, Björn**
  **223 61 Lund (SE)**
- **Holmdahl, Lena**
  **441 43 Alingsäs (SE)**

(72) Inventors:
- **Bengmark, Stig**
  **S-220 05 Lund (SE)**
- **LARSSON, Kare**
  **S-237 00 Bjärred (SE)**
- **Lindman, Björn**
  **223 61 Lund (SE)**
- **Holmdahl, Lena**
  **441 43 Alingsäs (SE)**

(74) Representative: **Petri, Stellan**
**Ström & Gulliksson AB**
**Box 41 88**
**203 13 Malmö (SE)**

(56) References cited:
**EP-A- 0 499 164**
**WO-A-90/10031**
**WO-A-91/12026**
**WO-A-96/32929**
**WO-A-97/07833**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 001 790 B1

## Description

The invention relates to a composition for lubricating and separating membranes from adjacent membranes or membranes from adjacent cells or tissues comprising a hydrophobized polymer. Preferably, the composition is used for preventing postsurgical adhesions, i.e. de novo adhesions which normally are caused by normal surgical manipulations and unintentional tissue injury.

Vital tissues such as blood vessels or organs are coated with mucous, serous, synovial and endothelial membranes so that they can function independently of each other. The peritoneum, i.e. the serous membrane lining the abdominopelvic walls and investing the viscera as well as the pericardial and pleural sacs, has a smooth surface and forms a double-layered sac which is closed in the male and is continuous with the mucous membrane of the uterine tubes in the female. The peritoneal, pericardial and pleural membranes consist of a single layer of mesothelial cells, which is covered with a thin film of peritoneal fluid., The components of the membranes as well as the covering layer of fluid have several functions, e.g. the lubrication of the enclosed organs, unrestricted mobility being provided.

The protective membrane is thus very thin and comprises a thin layer of connective tissue covered with a monolayer of mesothelial cells and only a few molecules thick layer of mainly phospholipids. When such a membrane is exposed to a physical, chemical or microbial challenge many potent substances harmful to the membrane are often released in response thereto. The structure and function of the membrane is consequently easily destroyed in connection with trauma, ischemia, and infection. After an irritation of the stress-sensitive membrane, e.g. only by the desiccation or abrasion of the membrane surfaces during surgery, it will rapidly be covered with a fibrin clot. Since the plasmiriogen activating activity (i.e. the fibrinolytic capacity) is reduced after trauma, the fibrin clots will later on become organized as fibrous adhesions, i.e. small bands or structures by which adjacent serous or synovial membranes adhere in an abnormal way. Surgical operations; infection or inflammation in those parts of the body which are coated with serous or synovial membranes can result in adhesive inflammation regardless of the size of the affected area. The adhesions between vital tissues are formed within the first few days following surgery trauma or infection and may be observed not only in particular portions of the body but in all vital tissues. Such adhesions between for example intestines or intestines and the abdominal wall are the result of the often unnoticed tissue damage as desiccation and they occur for various reasons including mechanical and chemical stimulations of vital tissues accompanying surgical manipulations, postoperative bacterial infection, inflammation or further complications.

Adhesion of vital tissues, large or small, may be observed in most surgical fields and the postsurgical adhesions obtained are the result of a natural wound healing response of tissue damage occurring during surgery. In fact, it has been reported that of all patients undergoing abdominal surgery at one hospital over a four-year period, 93 % were found to have adhesions from previous operations. In 1994 surgeons in the US performed about 400,000 operations in which adhesions were removed. It is thus of great interest to produce measures for preventing such postoperative adhesions between vital tissues.

In summary, all tissues having contact with each other have a tendency to adhere to each other by fibrinous and fibrous adhesions. This is so for surfaces covered with mesothelium, i.e. peritoneum, pleura and pericardium, surfaces covered with synovia, i.e. joints and tendon sheets, and for special surfaces such as the surfaces in the eye and the middle ear. Such adhesions also occur between different blood corpuscles and the inside of a blood vessel. The adhesions created by development of fibrin or connective tissue are unwanted since they result in a reduced function and a possible disease. In order to prevent this Nature has developed a system, whereby the surfaces are covered with layers of special components with the function of lubricating and separating tissues. These layers are, however, easily destroyed and this can have catastrophic consequences. While waiting after surgery for the body to produce new protective layers it is important to supply the corresponding protection from the outside in an effective way. Furthermore, it is important to prevent or reduce the infection and/or the inflammation obtained after surgery as well as the accompanying fibrin formation.

A number of methods for limiting the formation of surgical adhesion have been studied with some encouraging but often ambiguous results. However, most efforts made to avoid or reduce postoperative peritoneal adhesions have finally been abandoned. Among the methods used prevention of fibrin formation, reduction of fibrin formation, surface separation, and surgical techniques can be mentioned.

Numerous investigations have been carried out in which barriers are placed at a site of injury in order to prevent fibrin bridge formation between the injured tissue and neighboring organs. Such barriers include resorbable materials, such as enzymatically degradable oxidized regenerated cellulose, and slowly dissolving physiochemically crosslinked hydrogels of the Pluronic™ type.

Most methods of limiting postsurgical adhesion formation have also focused on providing wound separation by placing a material between the tissues. In addition, several types of viscous polymer solutions such as polyvinylpyrrolidone, sodium carboxymethyl cellulose, dextrans, and hyaluronic acid have been added before and/or at the end of surgery in order to control the wound healing events after the occurrence of the presumed tissue injuries. These solutions are supposed to act by increasing the lubrication and preventing the fibrin clots from adhering to other surfaces

or by mechanically separating damaged tissues while they heal.

In WO 9112026 the prevention of unwanted surgical adhesion between two tissue surfaces is obtained by interposing, between the surfaces, phospholipid in suspension or solution in a surgically acceptable carrier, which can include hyaluronic acid. A viscous solution of a phospholipid, propylene glycol and water is also described. Another approach of precoating for the prevention of peritoneal adhesion formation has been to exogenously supply membrane components by the addition of phospholipids as shown in SE 45793.

Less viscous polymer solutions have been used as a tissue protective coatings during surgery in order to maintain the natural lubricity of tissues and organs and to protect the enclosing membrane. Precoating for tissue protection and adhesion prevention includes coating tissues at the beginning of surgery before a significant tissue manipulation and irritation can occur and continues throughout the operation so that a protective coating can be maintained on the tissues.

US 5,366,964 shows a surgical viscoelastic solution for promoting wound healing, which is used in direct contact with cells undergoing wound healing. The solution is intended for cell protection and cell coating during surgery and comprises one or several polymeric components. Hydroxypropylmethylcellulose and chondroitin sulphate are supposed to lubricate the tissue, while sodium hyaluronate would provide viscoelastic properties to the solution.

Several agents of today for treating postsurgical adhesions contain hyaluronic acid. For example US 5,409,904 describes solutions which reduce cell loss and tissue damage intended for protecting endothelial cells during ophthalmic surgery. The compositions used are composed of a viscoelastic material comprising hyaluronic acid, chondroitin sulphate, modified collagen, and/or modified cellulose. In WO 9010031 a composition is described for preventing tissue adhesion after surgery containing dextran and hyaluronic acid act which substances are supposed to act synergistically. In WO 9707833 a barrier material for preventing surgical adhesions is shoiwn, which comprises benzyl esters or covalently crosslinked derivatives of hyaluronic acid.

A hyaluronic acid based agent manufactured by Pharmacia under the trademark Healon and originally intended as an intraocular instillation has been found to the most effective agent up to now. However, hyaluronic.acid is isolated from cock's crests and is thus very expensive as well as potentially allergenic even in small quantities and even more for large surfaces such as the peritoneum which has an area of about two $m^2$.

The above mentioned hydrophilic polymeric solutions are mainly based on the viscosity of the high molecular weight polymer which often increases dramatically with increasing concentration. The polymer in question is often a polysaccharide as in US 4,994,277, in which a viscoelastic gel of biodegradable xanthan gum in a water solution for preventing adhesions between vital tissues is described. However, the major disadvantage of these polymers when used for reducing for example peritoneal adhesions as protective coatings during surgery or surface separation agents after surgery is that they do not significantly reduce adhesions because of their short residence time in the peritoneal cavity. The result is that subsequent surgeries have to be performed on the patient.

Water-insoluble biocompatible compositions are shown in EP 0,705,878, which comprise a polyanionic polysaccharide combined with a hydrophobic bio-absorbable polymer.

EP 4,991,64 describes lubricating compositions comprising a hydrophilic polymer suspended in liquid carriers such as glycerol, propylene glycol, a fatty alcohol, a fatty acid, or polyethylene glycol.

WO 9319733 shows a moisturizer for epithelia which prevents abrasions and comprises bilayer particles of a hydrophobic adhesive polymer.

EP 713 708 shows a liquid polymer composition comprising a hydrophobic bioabsorbable polymer which will dissolve or disperse etc. in a hydrophilic liquid polymer. On contact with water the composition undergoes a phase separation resulting in the formation of an amorphous, liquid-crystalline or partially crystalline mass which is used in implants such as post surgical adhesion barriers.

WO 9508981 shows a contraceptive composition comprising a hydrophobically modified polysaccharide selected from cellulosics, chitosans and mixtures thereof, which may be used as a personal care lubricant.

Until now no product has exhibited a pronounced reduction of the adhesion formations in clinical trials. Neither does any of the above-mentioned documents eliminate the problems of currently applied formulations for preventing postoperative adhesion between organs or parts of organs caused by tissue manipulation.

The purpose of the present invention is to provide a composition for lubricating and separating tissues and biological membranes from adjacent membranes or membranes from adjacent cells or tissues. A composition according to the invention provides an effective temporary replacement of the normal protective layers by adhering to membrane surfaces by means of bioadhesion (mucoadhesion). An effective wound separation for preventing postsurgical adhesions between tissues is accomplished by the composition whereby the material of the composition provides a material which is not easily removed from its site of action. In order to achieve these purposes the method according to the invention has obtained the characterizing features of claim 1.

In order to further explain the invention reference is made to the accompanying drawings in which

FIG 1 is a diagram showing the difference in adhesion formation after surgery of mice between a control group

and two polymers according to the invention as well as a comparative compound; and
FIG 2 is a diagram showing the number of adhesion attachments to different intraabdominal structures after surgery as in FIG 1.

A composition according to the invention contains a high molecular weight water soluble cationic polymer having hydrophobic groups which can attach to membranes. The hydrophobic groups are covalently bonded to the polymer and are designed to penetrate into membranes which on their surfaces are covered with a mesothelial, synovial, or mucus layer. The mesothelium covers serous membranes such as peritoneum, pericardium, and pleura, and synovial membranes cover surfaces of joints and tendon sheets. Compositions according to the invention are especially adapted to be used on a mesothelial/serosal membrane. However, they can also be used for lubricating and preventing adhesions in other organs and/or carrying different compounds such as drugs or vaccines to body cavities or other organs.

The cationic polymers according to the invention have a hydrophilic backbone to which a low number of hydrophobic side chains, or tails, are covalently bonded. The polymers to be hydrophobized can be synthetic as well as natural.

The most preferred polymers to be modified, however, are different kinds of polysaccharides such as cellulose, starch, chitin, chitosan, and derivatives thereof. Of these cellulose is insoluble: derivatives thereof. Of these cellulose is insoluble in water so it is made soluble in an aqueous solution by grafting the polysaccharide with for example ethylene oxide and ethyl groups. A substitution with more hydrophobic substituents, such as methyl and hydroxypropyl, can also render such polymers water soluble. Thus, suitable cellulose derivatives are ethyl-hydroxyethyl cellulose, carboxymethyl cellulose, hydroxyethyl cellulose, methyl cellulose, and hydroxypropyl cellulose.

In such grafted hydrophobically modified water soluble polymers the number of hydrophobic groups in usually small. Preferably, 1-5 % of the monomer units are modified, i.e. below 5 mol% of hydrophobic side chains, or tails, are attached to the hydrophilic backbone. By combining a hydrophilic backbone with lipophilic pendant groups an affinity for the lipid/water interface is accomplished. Furthermore, solutions of hydrophobically modified polymers have been found to have higher viscosities than the solutions of their unmodified analogue. This feature of a polymer according to the invention assists in mechanically separating adjoining traumatized surfaces.

The hydrophobic modification can be performed with different kinds of substituents. Preferably, alkyl or alkylaryl groups are used. Most preferably, the substituents are alkyl chains with 10 to 18 carbon atoms. Compounds containing one or more benzene rings as well as cholesterol can also be used.

In compositions according to the invention cationic polymers are most effective in lubricating and separating membranes. An example of a water soluble cationic hydrophobically modified hydroxyethyl cellulose is shown below.

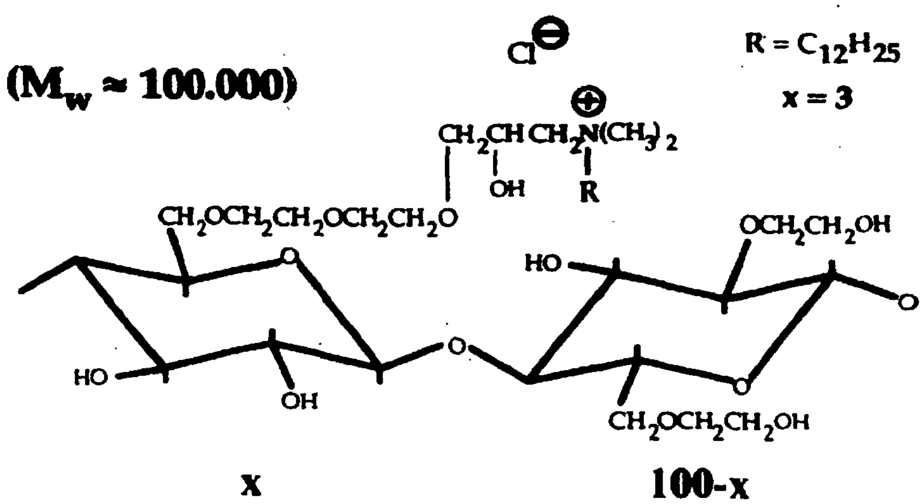

(I)

The preferred polysaccharides can be used separately or according to a further aspect of the invention be combined with certain lipids, such as polar lipids, to confer an enhanced hydrophobic effect to the polysaccharides. By increasing the hydrophobic bonding with such lipids the strength of the attachment between the polysaccharide and the peritoneal membrane is enhanced. This improved effect is especially obtained when the polar lipids are transformed into a vesicular state which then can form an association complex with the actual polysaccharide.

When used in compositions such lipids must be able to form vesicle dispersions, and the vesicles formed should be able to associate with the polysaccharides into a colloidal complex. In order to avoid any risk of toxicological effects being obtained phospholipids and galactolipids are preferred since these lipids are naturally occurring components in cell membranes.

Polysaccharides containing positive charges can be combined with phospholipids having at least 10 % (w/w) of negatively charged species which by mechanical dispersion processes are able to form kinetically stable vesicles with the polysaccharide in question. Other polysaccharides can be combined with neutral or zwitterionic polar lipids which similarly can form kinetically stable vesicles by mechanical dispersions with the polysaccharide in question.

An additional advantage of the present invention is that the treatment of the infection and/or the inflammation obtained after surgery as well as the accompanying, fibrin formation can be accomplished by a carrier of drugs being provided by means of what is called bioenhancing. In this embodiment of the invention the vesicles obtained can include different compounds and they can be used as carriers of these compounds to a specific site of action on a membrane in order to be released there. An example of such a compound can be a drug with the ability of preventing fibrin formation or enhancing fibrin dissolution, and there are several compounds available which inhibit the tissue plasminogen activator (tPA). Other suitable but not limiting compounds for site-specific delivery comprise drugs, vaccines, diagnostic agents etc. Preferably, a compound carried by the vesicles is an X-ray contrast agent, a drug enhancing fibrin degradation, e.g. tissue plasminogen activator (tPA), urokinase plasminogen activator (uPA), inhibitors of plasminogen activating inhibitors (PAI's), or a component that affect posttraumatic inflammations, e.g. agents that affect cytokines and growth factors, steroids and non-steroidal antiinflammatory drugs.

Furhter crosslinking of adjacent chains in the cationic polymers also increase the efficiacy of a composition according to the invention. For example, inositolhexaphosphate (IP6), also known as phytic acid, has exhibited unexpected good effects when combined with cationic polymers according to the invention. These positive effects are considered to be due to adjacent chains of the cationic polymer being crosslinked with IP6 which results in an enhanced barrier effect. Furthermore, the favourable tissue response of this polymer-IP6 combination takes advantage of the antioxidant functions and antiinflammatory effects of IP6.

An effective concentration of a hydrophobized cationic polymer in a composition according to the invention ranges from about 0.2 to about 5 weight%, preferably from about 0.5 to about 2 weight%, of the final liquid composition. The composition comprises polymers formulated with a pharmaceutically acceptable carrier according to well known, conventional methods. Preferably, buffered aqueous media are used which contain pharmaceutically acceptable salts and buffers.

A composition according to the invention can be added to the surgical area of the operation before and/or at the end of surgery. However, the compositions can for example also be added to the rinsing liquid for peritoneal dialysis in order to lubricate and prevent adhesions when used repeatedly.

When a composition according to the invention is added to exposed vital tissues of body cavities such as the mesothelium, synovia or mucus covered cavities a thin film is formed on the membranes, and a steric stabilization is achieved at the site of treatment. By the attachment of the cationic polymer to the membrane it is avoided that the film is removed by for example respiratory, cardiac, peristaltic, or other movements of the organs. It is important that the aqueous polymers as well as the lipids can be sterilized and that they are biologically acceptable. Preferably, the hydrophobized polymer material is a harmless biologically inert polymer which is not metabolized by the body, but a biodegradable material which dissolves over time in vital tissues can also be used.

The attachment of a polymer according to the invention to the membrane surface is accomplished by hydrophobic tails protruding from the polymer chain interact with the serous or mucous membrane. By this hydrophobic interaction a stabilizing effect is achieved in the membrane which facilitates its healing process. The polymers will thus remain in contact with the serous membranes for a considerably longer time than other polymers according to the state of the art.

By the use of a hydrophilic backbone with a low number, i.e. less than 5 mol%, of attached hydrophobic side chains, or tails, a structure is obtained which assists in keeping the membranes apart. The polymer forms an arch-like structure between the hydrophobic tails embedded in the biological membrane. In the space formed between these points the structure will be strengthened when the polymers are positively charged since they are attracted by the inherently negatively charged membranes. This attracting effect of opposite charged membranes has an additional advantage in increasing the lubricating and separating properties of the polymer by the friction between polymer and membrane being reduced.

Thus, a hydrophobic cationic polymer according to the invention can bind to a membrane by means of hydrophobic and/or electrostatic interactions.

**Examples.**

The present invention will now be explained more in detail with reference to the following examples.

Example 1.

A mixture of 20 g of phospholipids comprising 88 % (w/w) phosphatidylcholine, 8 % phosphatidylinositol, and 4 % phosphatidylglycerol was dissolved in ethanol and then freeze-dried in order to obtain molecular mixing. The purity of these commercial lipid samples (all from Sigma Chemical Co.) was 95 %. By using an Ultraturrax mixer the lipids were dispersed in 500 g of water which previously had been made isotonic by the addition of glycerol. When the dispersion became a semitransparent solution - which was stable when stored - a solution of 25 g of the polymer (I) in 500 g of an isotonic glycerol-water solution was added. The formulation was sterilized in glass ampoules.

Example 2.

A dispersion of 15 g of digalactosyl-diglycerides prepared from wheat gluten by chromatographic separation as described by Carlsson et al. (Cereal Chemistry 55 (1978) 168-179) in 500 g of physiological (isotonic) saline was prepared by means of ultrasonification. After the transformation of the dispersion from multi-bilayer liposomes to unibilayer vesicles, as evident from the semitransparent appearance and the lack of birefringence of the particles when viewed in the polarizing microscope, 500 g of a solution of 10 g of hydrophobically modified ethyl hydroxyethyl cellulose was added. The ultrasonification was allowed to continue for a few minutes in order to improve the polysaccharide hydrocarbon chain penetration into the vesicles. Samples were transferred to 10 g glass bottles and sterilized before use.

Example 3.

The formation of adhesions was quantified and the morphologic response in the tissue was studied after surgery of NMRI mice as an animal model. The effects of two polymers according to the invention were compared with a phospholipid as a reference.

Compound I (1% wt) was obtained as LM-200 from Bionord AB, Gothenburg, Sweden. Compound II (1% wt) was a hydrophobically modified ethyl-hydroxyethyl cellulose obtained from Akzo Nobel Surface Chemistry AB, Stenungsund, Sweden. As comparison sphingomyelin (95% wt) was used which was obtained from Swedish Dairy's Association, Research Department, Lund, Sweden. The sphingomyelin was diluted in sterile water to a final concentration of 1% wt. All compounds were kept refrigerated until used.

The animals - 142 female NMRI mice weighing 30-35 g-had free access to pellets and water before and after surgery. They were randomly allocated to different experimental groups.

One group (n=6) was sham-operated. Three groups were subjected to a short term treatment and three groups were given a prolonged treatment. The control groups consisted of animals which in a similar fashion received an equal volume of saline solution.

The short term treatment consisted of an intraperitoneal injection (i.p.) of either compound I (n=21), compound II (n=21) or the comparative compound (n=16) after closure of the abdominal cavity. The controls received saline only (n=16). The volume load was 0.75 ml (0.25 mg/g body weight). The groups receiving prolonged treatment were given an i.p. injection of either compound I (n=21), compound II (n=21) or the comparative compound (n=16) not only at the time of the operation but also on day 2 and day 3 postoperatively. The controls received saline only (n=16). In all cases the volume load was the same (0.75 ml). When the morphological analysis of tissue response was performed two animals from each group were sacrificed at day 1, 3, 5 and 7 for.

Surgery was performed after anesthesia induced and maintained by inhalation of methoxyflurane (Metofane®, Pitman Moore Inc., Mundelein, USA). Then the abdomen was shaved and opened with a 2.5 cm midline incision. The abdominal wall was held upwards with two forceps attached to *linea alba*, thereby exposing the interior. A 2 x 15 mm defect in the parietal peritoneum was created on each side of the lateral side wall by a sharp incision along a metal ruler followed by an excision with a pair of scissors. The lesion was closed with four 5/0 silk sutures, one at each end of the lesion and the other two at an equal distance between them. The midline incision was closed in two layers by a continuous 3/0 polyglycocolic mass closure (Dexon®, Davies and Geck, USA) according to well-known procedures.

At day 1, 3 and 5 post-operatively, two animals were sacrificed from each group by the administration of an overdose of isoflurane (Forene® , Abbott Laboratories Ltd., Queensborough, England) and biopsies were taken for morphological studies. Seven days after the operation the remaining animals were sacrificed. The abdominal cavity was widely opened with a U-shaped incision with its base to the right of the animal. The flap was then turned over to the right for displaying the parietal peritoneum of the anterior abdominal wall and the adhesions attached to it. The lesions previously induced to the peritoneum were identified by the suture lines. The evaluation was performed by measuring with a calliper the length of the original lesion and that of the individual adhesion attachments. The extent of the peritoneal defect covered with adhesions could then be calculated according to the following formula as previously described:

$$\text{Adhesions (\%)} = \frac{\text{Sum of attachments (mm)}}{\text{Excision site (mm) x 100}}$$

In addition, the location and the number of attachments were noted.

The injured area including the parietal peritoneum, the muscular layer and the skin was excised in order to study the morphology. The specimen was fixed in Bouin's solution for 24 hours, dehydrated and embedded in paraffin wax. Sections having a thickness of 5 μm were cut out and stained with haematoxylin and eosin.

In order to determine the significance of differences in adhesion formation between groups the values obtained and given as mean (SEM) were statistically evaluated by means of the non-parametric Mann-Whitney U test. A p value of less than 0.05 was considered to be statistically significant.

None of the animals had obtained any adhesions at the beginning of the operation. All animals developed adhesions, except for two animals which had received compound I as a prolonged treatment. No bowel obstruction or displayed signs of a generalized malaise had developed in any animal.

No adhesion formation was observed postoperatively in the sham-operated group except for the midline incision. As shown in FIG 1 there was no significant difference in adhesion formation between the control group [69.0 (4.5) %] and the group having obtained the comparative compound [69.7 (6.7) %, p=0.733] as a short term treatment. The group treated with compound II exhibited a significantly lesser adhesion formation [48.1 (5.1) %, p=0.012] than the control, as did the group treated with compound I [44.2 (6.3) %, p=0.013]. A repeated injection of the comparative compound two and three days postoperatively resulted in a reduction in adhesion formation [72.4 (4.5) %, p=0.049] compared with saline. Significantly less adhesions were seen in the groups receiving the long term treatment with compound II [47.6 (6.5) %, p=0.003] or compound I [24.4 (5.3) %, p<0.001] compared with the control group given three injections of NaCl [84.5 (6.2) %]. No difference in effect was obtained between the group receiving compound II as a short term treatment [48.1 (5.1) %] compared with the group receiving compound II as a prolonged treatment [47.6 (6.5) %, p=0.747). The adhesion reducing effect of compound I was significant with the short as well as the long term treatment. However, in contrast to compound II compound I was significantly more effective when given as a prolonged treatment [24.4 (5.3) %] compared with the short term treatment [44.2 (6.3) %, p=0.032] . The results are shown in FIG 1.

The number of adhesion attachments to different intraabdominal structures was also determined (FIG 2). The structures studied were omentum/pelvic fat bodies (PFB), bowel (B), or other (O, predominantly liver). As shown in FIG 2 there was no significant effect of compound I compared with the control on the number of adhesions when given as a short term treatment (PFB, p=0.098; B, p=0.114; O, p=0.717). The same result was obtained with compound II (PFB, p=0.205; B, p=0.103; O, p=0.717). The comparative compound sphingomyelin reduced the number of attachments to omental structures but not to the bowel or to other parts of the abdominal cavity (PFB, p=0.049; B, p=0.434; O, p=0.342).

When compound I was given in three consecutive doses a significant reduction of the number of attachments to both the omentum (p=0.023) and the bowel (p<0.001) was obtained, but not to other structures (p=0.147). Compound II reduced the number of attachments to the bowel and other structures (B, p= 0.004; 0, p=0.032) but not to omentum/pelvic fat bodies (p=0.596). When given as a long term treatment the comparative compound exhibited no reducing effect at all on the number of attachments to any of the structures involved (PFB, p=0.720; B, p=0.091; O, p=0.888). When compound I was given as a prolonged treatment and compared with that group which had received the short term treatment significantly fewer adhesions were obtained to both omentum/pelvic fat bodies and bowel (p=0.023; p=0.032) but not to other structures (p=0.549). The data obtained are summarized in FIG 2.

When the tissue response was studied an inflammatory reaction could be seen at the site of adhesion formation. This was observed not only at the attachment site and in the abdominal wall but also in the adherent non-injured tissue. On day 1 post-surgery polymorphonuclear neutrophils dominated and later macrophages were abundant. A uniform morphologic appearance was seen on specimens obtained from all groups, and there was no difference between the treatment groups and the control group. Furthermore, no difference was obtained between the groups given short term treatment and long term treatment.

## Claims

**1.** A liquid composition for lubricating and separating tissues and biological membranes from adjacent membranes or membranes from adjacent cells or tissues, which comprises a biologically acceptable hydrophobized polysaccharide, **characterized in that** said hydrophobized polysaccharide is a cationic water soluble hydrophobically modified polysaccharide or polysaccharide derivative, which has a hydrophilic backbone with hydrophobic groups covalently bonded thereto, said hydrophobized polysaccharide being capable of binding to any one of said membranes by means of hydrophobic and/or electrostatic interactions, said membrane having on its surface one of a

mesothelial, synovial, and mucus layer.

2. The liquid composition as in claim 1, **characterized in that** said polysaccharide is chitosan.

3. The liquid composition as in claim 1, **characterized in that** said polysaccharide derivative is a cellulose derivative.

4. The liquid composition as in claim 3, **characterized in that** said cellulose derivative is ethyl-hydroxyethyl cellulose, carboxymethyl cellulose, hydroxyethyl cellulose, methyl cellulose, or hydroxypropyl cellulose.

5. The liquid composition as in any of claims 1-4, **characterized in that** said hydrophobic groups are alkyl groups, alkylaryl groups, compounds containing one or more benzene rings, or cholesterol.

6. The liquid composition as in claim 5, **characterized in that** said alkyl groups have 10 to 18 carbon atoms.

7. The liquid composition as in claim 1, **characterized in that** the number of said hydrophobic groups is on average 1-5% of the repeating units in said hydrophilic backbone.

8. The liquid composition as in any of claims 1-7, **characterized in that** said hydrophobized polysaccharide has been combined with inositolhexaphosphate in order to improve the barrier effect.

9. The liquid composition as in any of claims 1-7, **characterized in that** said hydrophobized polysaccharide has been combined with a lipid in order to improve the bonding to said membrane.

10. The liquid composition as in claim 9, **characterized in that** said lipid is able to form a vesicle dispersion that can associate with said hydrophobized polysaccharide into a colloidal complex.

11. The liquid composition as in claim 9 or 10, **characterized in that** said lipid is a phospholipid or a galactolipid.

12. The liquid composition as in claim 11, **characterized in that** said phospholipid has at least 10 % (w/w) of negatively charged species.

13. The liquid composition as in any of claims 9-11, **characterized in that** said lipid is a neutral or a zwitterionic polar lipid, which can form kinetically stable vesicles with said hydrophobized polysaccharide.

14. Use of a liquid composition as in any of claims 10-13 for the manufacturing of a medicament for the delivery of drugs, vaccines, diagnostic agents to a specific site of action on a membrane in order to be released therefrom.

**Patentansprüche**

1. Flüssige Zusammensetzung zur Schmierung und Trennung von Geweben und biologischen Membranen von angrenzenden Membranen oder Membranen von angrenzenden Zellen oder Geweben, welche Zusammensetzung ein biologisch akzeptierbares hydrophobiertes Polysaccharid umfasst, **dadurch gekennzeichnet, dass** das hydrophobierte Polysaccharid ein kationisches wasserlösliches hydrophobisch modifiziertes Polysaccharid oder Polysaccharidderivat ist, welches einen hydrophilen Träger mit kovalent daran angebundenen hydrophoben Gruppen aufweist, wobei das hydrophobierte Polysaccharid in der Lage ist, an eine der genannten Membranen mittels hydrophober und/oder elektrostatischer Wechselwirkungen anzubinden, wobei die Membran an ihrer Oberfläche eine Mesothelial-, Synovial- oder Mucus-Schicht aufweist.

2. Flüssige Zusammensetzung gemäß Anspruch 1, **dadurch gekennzeichnet, dass** das Polysaccharid Chitosan ist.

3. Flüssige Zusammensetzung gemäß Anspruch 1, **dadurch gekennzeichnet, dass** das Polysaccharidderivat ein Zellulosederivat ist.

4. Flüssige Zusammensetzung gemäß Anspruch 3, **dadurch gekennzeichnet, dass** das Zellulosederivat Ethyl-Hydroxiethyl-Zellulose, Carboximethyl-Zellulose, Hydroxiethyl-Zellulose, Methyl-Zellulose oder Hydroxipropyl-Zellulose ist.

5. Flüssige Zusammensetzung gemäß einem der Ansprüche 1-4, **dadurch gekennzeichnet, dass** die hydrophoben Gruppen Alkyl-Gruppen, Alkaryl-Gruppen, Gemische, welche einen oder mehrere Benzolringe beinhalten, oder Cholesterol sind.

6. Flüssige Zusammensetzung gemäß Anspruch 5, **dadurch gekennzeichnet, dass** die Alkylgruppen 10 bis 18 Kohlenstoffatome aufweisen.

7. Flüssige Zusammensetzung gemäß Anspruch 1, **dadurch gekennzeichnet, dass** die Anzahl hydrophober Gruppen durchschnittlich 1-5% der sich wiederholenden Einheiten des hydrophilen Trägers beträgt.

8. Flüssige Zusammensetzung gemäß einem der Ansprüche 1-7, **dadurch gekennzeichnet, dass** das hydrophobierte Polysaccharid an Inositolhexaphosphat gebunden ist, um die Sperrwirkung zu verbessern.

9. Flüssige Zusammensetzung gemäß einem der Ansprüche 1-7, **dadurch gekennzeichnet, dass** das hydrophobierte Polysaccharid an ein Lipid gebunden ist, um die Verbindung mit der Membran zu verbessern.

10. Flüssige Zusammensetzung gemäß Anspruch 9, **dadurch gekennzeichnet, dass** das Lipid in der Lage ist, eine Vesikeldispersion zu bilden, welche mit dem hydrophobierten Polysaccharid zu einem Kolloidkomplex assoziieren kann.

11. Flüssige Zusammensetzung gemäß Anspruch 9 oder 10, **dadurch gekennzeichnet, dass** das Lipid ein Phospholipid oder ein Galaktolipid ist.

12. Flüssige Zusammensetzung gemäß Anspruch 11, **dadurch gekennzeichnet, dass** das Phospholipid wenigstens 10% (w/w) negativ geladene Arten umfasst.

13. Flüssige Zusammensetzung gemäß einem der Ansprüche 9-11, **dadurch gekennzeichnet, dass** das Lipid ein neutrales oder zwitterionisches polares Lipid ist, welches kinetisch stabile Vestikel mit dem hydrophobierten Polysaccharid bilden kann.

14. Anwendung einer flüssigen Zusammensetzung gemäß einem der Ansprüche 10-13 zur Herstellung eines Medikamentes zur Verabreichung von Arzneimitteln, Impfstoffen, oder diagnostischen Mitteln zu einer spezifischen Wirkungsstelle auf einer Membran, um von dort freigesetzt zu werden.

## Revendications

1. Composition liquide destinée à la lubrification et à la séparation de tissus et de membranes biologiques des membranes adjacentes ou des membranes de cellules ou tissus adjacents, qui comprend un polysaccharide rendu hydrophobe biologiquement acceptable, **caractérisée en ce que** ledit polysaccharide rendu hydrophobe est un polysaccharride ou dérivé de polysaccharide cationique modifié pour le rendre hydrophobe soluble dans l'eau, qui a un squelette hydrophile avec des groupes hydrophobes liés par covalence sur celui-ci, ledit polysaccharide rendu hydrophobe étant capable de liaison sur chacune desdites membranes au moyen d'interactions hydrophobes et/ou électrostatiques, ladite membrane ayant sur sa surface une couche mésothéliale, synoviale et muqueuse.

2. Composition liquide selon la revendication 1, **caractérisée en ce que** ledit polysaccharide est le chitosane.

3. Composition liquide selon la revendication 1, **caractérisée en ce que** ledit dérivé de polysaccharide est un dérivé de cellulose.

4. Composition liquide selon la revendication 3, **caractérisée en ce que** ledit dérivé de cellulose est l'éthyl-hydroxyéthyl-cellulose, la carboxyméthyl-cellulose, l'hydroxyéthyl-cellulose, la méthyl-cellulose, ou une hydroxypropyl-cellulose.

5. Composition liquide selon l'une des revendications 1 à 4, **caractérisée en ce que** lesdits groupes hydrophobes sont des groupes alkyle, des groupes alkylaryle, des composés contenant un ou plusieurs noyaux benzéniques, ou du cholestérol.

6. Composition liquide selon la revendication 5, **caractérisée en ce que** lesdits groupes alkyle ont de 10 à 18 atomes de carbone.

7. Composition liquide selon la revendication 1, **caractérisée en ce que** le nombre desdits groupes hydrophobes est en moyenne 1-5 % des unités répétitives dans ledit squelette hydrophile.

8. Composition liquide selon l'une des revendications 1 à 7, **caractérisée en ce que** ledit polysaccharide rendu hydrophobe a été combiné avec l'hexaphosphate d'inositol afin d'améliorer l'effet de barrière.

9. Composition liquide selon l'une des revendications 1 à 7, **caractérisée en ce que** ledit polysaccharide rendu hydrophobe a été combiné avec un lipide afin d'améliorer la liaison à ladite membrane.

10. Composition liquide selon la revendication 9, **caractérisée en ce que** ledit lipide est capable de former une dispersion des vésicules qui peut s'associer avec ledit polysaccharide rendu hydrophobe en un complexe colloïdal.

11. Composition liquide selon la revendication 9 ou 10, **caractérisée en ce que** ledit lipide est un phospholipide ou un galactolipide.

12. Composition liquide selon la revendication 11, **caractérisée en ce que** ledit phospholipide a au moins 10 % (en poids) d'espèces chargées négativement.

13. Composition liquide selon l'une des revendications 9 à 11, **caractérisée en ce que** ledit lipide est un lipide neutre ou un lipide polaire amphotère, qui peut former des vésicules cinétiquement stables avec ledit polysaccharide rendu hydrophobe.

14. Utilisation d'une composition liquide selon l'une quelconque des revendications 10 à 13 pour la fabrication d'un médicament destiné à l'administration de médicaments, vaccins, agents de diagnostic en un site d'action spécifique sur une membrane afin d'en être libérés.

FIG 1

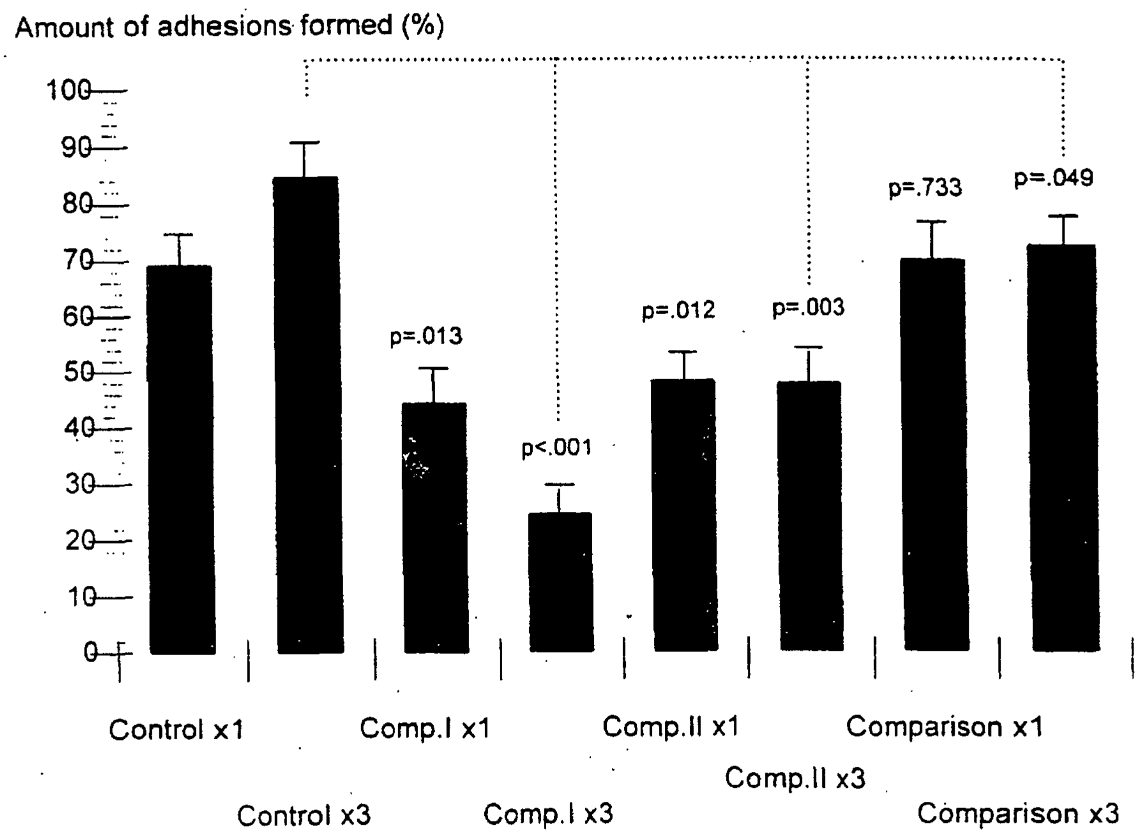
Amount of adhesions formed (%)
100
90
80
70
60
50
40
30
20
10
0
p=.013
p<.001
p=.012
p=.003
p=.733
p=.049
Control x1
Control x3
Comp.I x1
Comp.I x3
Comp.II x1
Comp.II x3
Comparison x1
Comparison x3

FIG 2

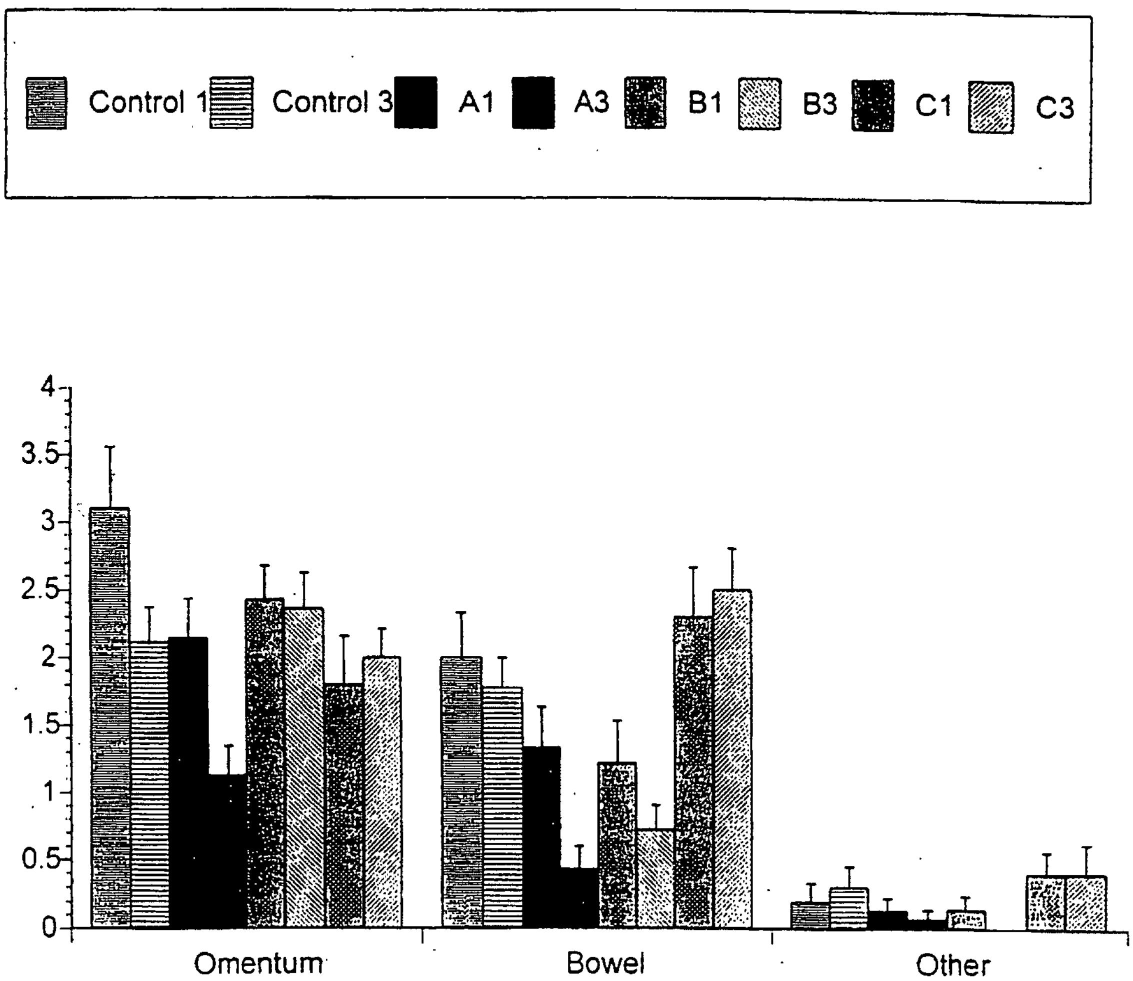
Control 1
Control 3
A1
A3
B1
B3
C1
C3
4
3.5
3
2.5
2
1.5
1
0.5
0
Omentum
Bowel
Other